# FASCICULE DE BREVET EUROPEEN

(11) **EP 3 471 634 B1**
(45) Date de publication et mention de la délivrance du brevet: **05.08.2020**
(21) Numéro de dépôt: 17740044.7
(22) Date de dépôt: 20.06.2017
(51) Int. Cl.: A61B 17/70, A61B 17/86, A61B 17/88

(54) **IMPLANT MEDICAL POUR INJECTION CIBLEE**
MEDIZINISCHES IMPLANTAT ZUR GEZIELTEN INJEKTION
MEDICAL IMPLANT FOR TARGETED INJECTION

(30) Priorité: 21.06.2016 FR 1600992
(43) Date de publication de la demande: 24.04.2019
(73) Titulaire: Innoprod Medical, 31830 Plaisance du Touch (FR)
(72) Inventeur: LEGAY, Philippe Alain Lucien Fernand, 76530 Yville sur Seine (FR); DESCHAMPS, Frédèric, 75015 Paris (FR); PEYRE, Frédéric, F-31530 Lasserre (FR)
(74) Mandataire: Icosa
(86) Numéro de dépôt international: PCT/FR2017/000126
(87) Numéro de publication internationale: WO 2017/220873

(56) Documents cités:
- EP-A1- 1 110 510
- WO-A1-2011/063240
- WO-A1-2014/149746
- US-A1- 2011 040 337
- US-A1- 2011 060 373

## Description

La présente invention concerne un implant médical permettant l'injection ciblée d'un matériau liquide ou pâteux dans une cavité osseuse, favorisant ainsi le comblement et/ou la consolidation, sans risque de contraindre tout nerf, et/ou toute autre partie molle, pouvant être présent à proximité du site d'injection.

L'art antérieur propose divers matériels employés en ostéosynthèse, clous et vis, canulés de façon à être guidés lors de la pose.

Ces différents matériels (par example les demandes de brevet US 2011/060373, WO 2014/149746 A1 ou EP 1110510 A1) peuvent être utilisés pour conduire un ciment acrylique, par l'intermédiaire de la lumière, mais en aucun cas ne permettent de cibler le ou les sites d'injection.

Les matériels utilisés actuellement par les praticiens n'étant pas dédiés à cette technique,
soit ne permettent pas d'injecter, obligeant ainsi à effectuer des injections différenciées, en pratiquant d'autres voies d'abord, donc des actions supplémentaires préjudiciables pour le patient,
soit ne permettent pas d'éviter les nerfs ou parties molles passant dans la cavité, à proximité de l'implant, donnant un risque de douleurs au patient.

La présente invention vise à donner la possibilité à l'intervenant de résoudre ces problèmes, par l'utilisation d'un implant modulable, qui permet, grâce à un implant principal et des implants complémentaires, de répondre aux besoins de la pratique, et notamment de cibler les zones, déterminées préalablement, ne devant pas recevoir de produit de comblement ou de consolidation.

Ce but est atteint grâce à un implant principal, pouvant être un clou, une broche ou une vis.

Avantageusement, l'implant ici décrit est un ensemble composé d'une vis à os appelée à recevoir des implants complémentaires, caractérisé
- en ce que ladite vis à os présente trois parties dont la partie centrale, appelée corps, peut être de section cylindrique, carrée ou polygonale. De préférence, selon l'invention, la section du corps de la vis à os est hexagonale de façon à offrir suffisamment de faces planes,
- en ce que ledit corps hexagonal comporte des trous cylindriques le traversant de part en part, jusqu'à l'axe longitudinal de la vis à os,
- en ce que les trous cylindriques peuvent être revêtus d'un taraudage mécanique,
- en ce que l'axe longitudinal de la vis à os est évidé, de manière à ce que les trous cylindriques débouchent dans cet évidement appelé lumière,
- en ce que la partie proximale renferme une empreinte femelle contiguë à une chambre d'injection pouvant s'étendre sur toute la longueur du corps de ladite vis appelée à recevoir une canule spécifique d'une instrumentation dédiée,
- en ce que lesdits implants complémentaires sont des bouchons, préférentiellement des micros vis, appelés à boucher un, plusieurs, ou tous les trous taraudés de l'implant principal,
- en ce que lesdits implants complémentaires sont composés de deux parties, dont la partie proximale est une tige, appelé manchon d'insertion, et la partie distale est un bouchon, utilement une micro vis à filetage mécanique de même pas que le taraudage des trous cylindriques du corps de l'implant principal,
- en ce que ladite micro vis est d'épaisseur égale à la profondeur de chacun des trous taraudés du corps de la vis principale,
- en ce que le manchon d'insertion et la micro vis distale sont séparés par une zone sécable,
- en ce que le manchon d'insertion, servant à positionner la micro vis, se détache en cisaillement, ou en flexion, au niveau de la zone sécable, en fin de serrage.

La vis à os comporte un filetage à chaque extrémité, lesdits filetages étant de pas différents,
Avantageusement, le filetage proximal peut être de pas plus étroit que le filetage distal de manière à effectuer une légère compression au serrage.

Le manchon d'insertion de chaque implant complémentaire peut être cylindrique ou de section carrée ou polygonale,
Avantageusement, ledit manchon d'insertion peut comporter, en son extrémité, une partie étranglée et plane permettant une meilleure préhension pour effectuer la mise en place et la désolidarisation de la micro vis.

L'invention sera bien comprise et d'autres caractéristiques et avantages de celle-ci apparaîtront en référence aux dessins schématiques annexés, représentant, à titre d'exemple non limitatif, une forme de réalisation de l'implant pour injection ciblée qu'elle concerne.
- la figure 1 représente une vue de l'implant principal,
- la figure 2 est une coupe longitudinale de l'implant principal,
- la figure 3 représente une vue d'un implant complémentaire,
- la figure 4 représente un implant principal, sur lequel a été positionné un implant complémentaire, après rupture du manchon d'insertion.

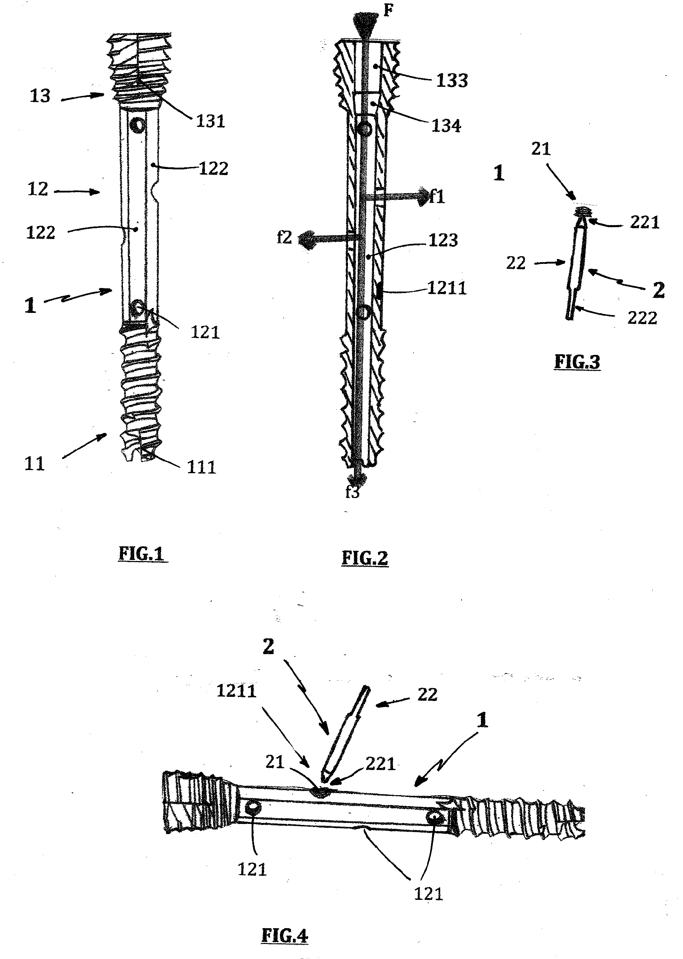

La figure 1 représente une vue de l'implant principal 1 composé d'une portion filetée distale 11, d'un corps intermédiaire 12 et d'une portion filetée proximale 13, l'ensemble étant monobloc.

Le corps intermédiaire 12, de section hexagonale s'inscrivant dans un cercle de même diamètre que la portion filetée 11, est perforé d'un nombre variable, en fonction de sa longueur, de trous préférentiellement taraudés 121 sur ses faces planes 122.

La portion filetée distale 11 et la portion filetée proximale 13 sont dotées d'un principe auto-perforant et auto-taraudant, respectivement 111 et 131.

La figure 2 est une coupe longitudinale de l'implant principal 1, présentant un évidemment 123, nommé lumière, communiquant avec les trous taraudés 121, afin de canaliser la conduction d'un matériau liquide ou pâteux, injecté au niveau de la chambre d'injection 134, pouvant s'étendre sur toute la longueur du corps de ladite vis, contigu à l'empreinte 133 appelée à coopérer avec un outil de mise en place, à l'extérieur de l'implant principal 1.

La flèche F montre le cheminement du matériau liquide ou pâteux dans la lumière 123 et, à titre d'exemple, ses sorties latérales f1, f2, par les trous taraudés 121 et par le bout de la lumière 123 représenté par f3, ainsi que le blocage du même matériau liquide ou pâteux, stoppé par le bouchage qu'opère une micro vis 21 coopérant avec un trou taraudé 121 en position 1211.

La figure 3 représente la vue d'un implant complémentaire 2, composé d'un bouchon, préférentiellement une micro vis 21 attenante à un manchon d'insertion 22 par l'intermédiaire d'une zone sécable 221.

Chacune desdites micros vis 21 est appelée à boucher un trou taraudé 121, préalablement déterminé, du corps 12 de l'implant principal 1 pour empêcher la diffusion du matériau liquide ou pâteux à cet endroit précis.

Avantageusement, le manchon 22 peut se terminer par un étranglement 222 permettant une meilleure préhension pour insérer la micro vis 21 dans les trous taraudés 121 et la désolidariser en fin de serrage.

La figure 4 représente une réalisation de l'invention composée d'un implant principal 1 dont un trou taraudé 121 est bouché par l'action d'une micro vis 21, détachée de son manchon d'insertion 22, après rupture de la zone sécable 221, pour stopper la diffusion du matériau liquide ou pâteux sur un site prédéterminé, tel que représenté, à titre d'exemple, en position 1211.

Comme cela apparaît de tout ce qui précède, l'invention fournit un implant, notamment d'ostéosynthèse, présentant, par rapport aux vis homologues de la technique antérieure ou empruntée à d'autres disciplines, l'avantage déterminant de permettre de cibler l'injection d'un matériau liquide ou pâteux dans une cavité osseuse, en particulier pour réaliser un comblement ou une consolidation, dans les meilleures conditions, et en évitant tout risque de contraindre tout nerf, et/ou toute autre partie molle, pouvant être présent à proximité du site d'injection.

Il va de soi que l'invention n'est pas limitée à la forme de réalisation décrite ci-dessus à titre d'exemple, mais qu'elle s'étend à tous les matériels et toutes les formes de réalisations couvertes par les revendications ci-après annexées.

## Revendications

1. Implant médical pour injection ciblée composé d'un implant principal (1) et d'un ou plusieurs implants complémentaires (2),
**caractérisé en ce que** l'implant principal (1) comporte trois parties dont la partie centrale appelée corps (12) de section polygonale, préférentiellement hexagonale, offre des faces planes (122) sur toute sa périphérie, lesquelles sont perforées jusqu'à son axe longitudinal évidé en lumière (123) d'orifices, préférentiellement de trous taraudés (121) appelés à être bouchés par une ou plusieurs micro vis (21) provenant des implants complémentaires (2), ou à rester ouverts, et que la partie proximale (13) renferme une chambre d'injection (134) pouvant s'étendre sur toute la longueur du corps de ladite vis, appelée à recevoir une canule spécifique d'une instrumentation dédiée ;
**en ce que** chaque implant complémentaire (2) est constitué d'un bouchon, utilement une micro vis (21) appelée à boucher l'un des trous taraudés (121) de l'implant principal (1), séparée d'un manchon d'insertion (22) par une zone sécable (221).

2. Implant médical pour injection ciblée selon la revendication 1, **caractérisé en ce que** les micro vis (21) sont positionnées de façon à boucher un, plusieurs ou tous les trous taraudés (121) pour stopper la diffusion d'un matériau, liquide ou pâteux, en des sites ciblés et préalablement déterminés, tel que présenté en position (1211).

3. Implant médical pour injection ciblée selon les revendications 1 et 2, **caractérisé en ce que** la micro vis (21) présente zone sécable (221) permettant de désolidariser le manchon d'insertion (22), par effet de cisaillement ou en flexion, après vissage de ladite micro vis (21) dans l'un des trous taraudés (121) de l'implant principal (1).

4. Implant médical pour injection ciblée selon les revendications 1 à 3, **caractérisé en ce que** le manchon d'insertion (22) des implants complémentaires (2), peut se terminer par un étranglement (222) permettant une meilleure préhension pour insérer la micro vis (21) dans les trous taraudés (121) et la désolidariser en fin de serrage.

5. Implant médical pour injection ciblée selon la revendication 1, **caractérisé en ce que** les extrémités de l'implant principal sont des portions filetées (11,13), chacune étant dotée de principe auto perforant et auto taraudant (111,131).

## Patentansprüche

1. Medizinisches Implantat zur gezielten Injektion, bestehend aus einem Hauptimplantat (1) und einem oder mehreren Komplementärimplantaten (2), **dadurch gekennzeichnet, dass** das Hauptimplantat (1) drei Teile umfasst, wobei der zentrale Teil, Körper (12) genannt, von polygonalem, bevorzugt hexagonalem Querschnitt, flache Seiten (122) auf seiner gesamten Peripherie bietet, welche bis zu seiner lichtverschlossenen Längsachse (123) von Öffnungen perforiert sind, bevorzugt von Gewindelöchern (121), dafür vorgesehen, von einer oder mehreren von den Komplementärimplantaten (2) stammenden Mikroschrauben (21) verschlossen zu werden oder offen zu bleiben, und dass der proximale Teil (13) eine Injektionskammer (134) birgt, die sich über die gesamte Länge des Körpers der Schraube erstrecken kann, dafür vorgesehen, eine spezifische Kanüle einer dezidierten Instrumentation aufzunehmen;
dadurch, dass jedes Komplementärimplantat (2) aus einem Verschluss besteht, zweckdienlicherweise einer Mikroschraube (21), vorgesehen, um eines der Gewindelöcher (121) des Hauptimplantats (1) zu verschließen, getrennt durch eine Einführungsmuffe (22) über einen abtrennbaren Bereich (221).

2. Medizinisches Implantat zur gezielten Injektion nach Anspruch 1, **dadurch gekennzeichnet, dass** die Mikroschrauben (21) solcherart positioniert sind, dass sie ein, mehrere oder alle Gewindelöcher (121) verschließen, um an vorher festgelegten Zielorten, wie in Position (1211) dargestellt, die Diffusion eines flüssigen oder pastösen Materials zu stoppen.

3. Medizinisches Implantat zur gezielten Injektion nach den Ansprüchen 1 und 2, **dadurch gekennzeichnet, dass** die Mikroschraube (21) einen trennbaren Bereich (221) aufweist, der es erlaubt, nach dem Schrauben der Mikroschraube (21) in eines der Gewindelöcher (121) des Hauptimplantats (1) die Einführungsmuffe (22) durch Scherungseffekt oder biegend zu desolidarisieren.

4. Medizinisches Implantat zur gezielten Injektion nach den Ansprüchen 1 bis 3, **dadurch gekennzeichnet, dass** die Einführungsmuffe (22) der Komplementärimplantate (2) in einem Engpass (222) enden kann, der einen besseren Griff erlaubt, um die Mirkoschraube (21) in die Gewindelöscher (121) einzuführen sie am Ende des Festziehens zu desolidarisieren.

5. Medizinisches Implantat zur gezielten Injektion nach Anspruch 1, **dadurch gekennzeichnet, dass** die Extremitäten des Hauptimplantats Gewindeabschnitte (11, 13) sind, wobei jeder mit selbst-perforierendem und selbst-gewindebohrendem (111, 113) Prinzip ausgestattet ist.

## Claims

1. Medical implant for targeted injection composed of a main implant (1) and one or more complementary implants (2), **characterized in that** the main implant (1) has three parts, the central part of which is called the body (12) of polygonal section, preferably hexagonal, offers planar faces (122) over its entire periphery, which are perforated to its longitudinal axis hollowed in light (123) of orifices, preferably tapped holes (121) called to be plugged by one or more micro screws (21) coming from complementary implants (2), or to remain open, and that the proximal part (13) contains an injection chamber (134) which can extend over the entire length of the body of said screw, called to receive a specific cannula of dedicated instrumentation; **in that** each complementary implant (2) consists of a plug, usefully a micro screw (21) called to plug one of the tapped holes (121) of the main implant (1), separated by a sleeve insertion (22) by a breakable zone (221).

2. Medical implant for targeted injection according to claim 1, **characterized in that** the micro screws (21) are positioned so as to block one, several or all of the tapped holes (121) to stop the diffusion of a material, liquid or pasty, in targeted and previously determined sites, as presented in position (1211).

3. Medical implant for targeted injection according to claims 1 and 2, **characterized in that** the micro-screw (21) has a breakable zone (221) enabling the insertion sleeve (22) to be separated, by shearing or bending effect, after screwing of said micro screw (21) in one of the threaded holes (121) of the main implant (1).

4. Medical implant for targeted injection according to claims 1 to 3, **characterized in that** the insertion sleeve (22) of the complementary implants (2), can end in a constriction (222) allowing a better grip for inserting the micro screw (21) in the tapped holes (121) and detach it at the end of tightening.

5. Medical implant for targeted injection according to claim 1, **characterized in that** the ends of the main implant are threaded portions (11, 13), each being provided with self-perforating and self-tapping principle (111, 131).
